# EUROPÄISCHE PATENTSCHRIFT

(11) **EP 0 620 827 B1**
(45) Veröffentlichungstag und Bekanntmachung des Hinweises auf die Patenterteilung: **21.01.1998**
(21) Anmeldenummer: 92921668.7
(22) Anmeldetag: 20.10.1992
(51) Int. Cl.: C07K 16/00, A61K 39/395

(54) **VERFAHREN ZUR HERSTELLUNG VON NATIVEM, INTRAVENÖS VERABREICHBAREM, STABILISIERTEM IMMUNGLOBULIN**
PROCESS FOR PRODUCING NATIVE,INTRAVENOUSLY ADMINISTRABLE STABILISED IMMUNOGLOBIN
PROCEDE DE PREPARATION D'IMMUNOGLOBULINE NATIVE STABILISEE A ADMINISTRATION INTRAVEINEUSE

(43) Veröffentlichungstag der Anmeldung: 26.10.1994
(73) Patentinhaber: HILLGER, Karl, D-06846 Dessau (DE); SETTER, Jürgen, D-26419 Schorten (DE); SCHWEIGLE, Bernard, D-06862 Meinsdorf (DE)
(72) Erfinder: HILLGER, Karl, D-06846 Dessau (DE); SETTER, Jürgen, D-26419 Schorten (DE); SCHWEIGLE, Bernard, D-06862 Meinsdorf (DE)
(74) Vertreter: Brehm, Hans-Peter, Dr., Dipl.-Chem.
(86) Internationale Anmeldenummer: EP9202405
(87) Internationale Veröffentlichungsnummer: WO9409038

(56) Entgegenhaltungen:
- EP-A- 0 240 856
- DE-A- 4 115 910
- FR-A- 2 513 640

## Beschreibung

Die Erfindung betrifft ein Verfahren zur Herstellung von nativem, intravenös verabreichbarem, stabilisiertem Immunglobulin (IgG). Nativ bezeichnet in diesem Zusammenhang ein chemisch nicht verändertes und enzymatisch nicht abgebautes Immunglobulin, das eine unveränderte Antikörperaktivität aufweist. Ein derartiges, intravenös verabreichbares Immunglobulin bildet ein wertvolles Mittel zur Prophylaxe und zur Behandlung von infektiösen Krankheiten und Antikörpermangelzuständen bei Mensch und Tier. Zur gefahrlosen intravenösen Applikation ist wichtig, daß derartige Immunglobuline keine nachweisbare antikomplementäre Aktivität aufweisen, die auf polymere Immunglobulinaggregate zurückführbar ist. Durch Zusatz von Stabilisierungsmitteln wie beispielsweise Albumin, Zucker, Glukose und/oder anderen Polyglykolen, sowie Polyolen wie etwa Mannit wird verhindert, daß das polymerfreie, applizierbare IgG-Präparat bei längerer Lagerung erneut polymere IgG-Aggregate bildet.

Mehr im einzelnen betrifft die Erfindung ein Verfahren zur Herstellung von nativem, intravenös verabreichbarem, stabilisiertem Immunglobulin (IgG), wobei bei einer Temperatur unterhalb 8° C
a) unter Maßnahmen zur schonenden Gesamtglobulinfällung aus Serum, Plasma oder Plazenten ein IgG enthaltender Gesamtglobulinniederschlag ausgefällt und abgetrennt wird, der weniger als 1 Gew.-% Polymere enthält,
b) dieser Gesamtglobulinniederschlag bei schwach saurem pH mit einer schwach ionischen, wässrigen, alkoholischen Lösung behandelt wird, und
c) die danach erhaltene IgG-Lösung vom alkoholischen Lösungsmittel befreit, auf eine gewünschte Proteinkonzentration sowie eine isotonische Salzkonzentration und einem physiologisch verträglichen pH Wert eingestellt und durch Zugabe von Stabilisierungsmittel(n), Sterilisation und gegebenenfalls Virusinaktivierung/-Elimination, zu einem applizierbaren Präparat weiterverarbeitet wird.

Verfahren dieser Art sind bekannt. So offenbart das Dokument EP 0 085 747 B2 ein Verfahren zur Herstellung von intravenös verabreichbarem Immunglobulin zur Verstärkung der Immunabwehr des menschlichen Organismus, bei welchem die wesentliche Reinigungsstufe, d.h. die Erzeugung eines geklärten Extraktes, mittels Chromatographie an einem Ionenaustauscher durchgeführt wird. Das als Ausgangsmaterial eingesetzte native Immunglobulin wird in einer Proteinkonzentration von 20 bis 40 mg/ml und einem pH von 4,0 bis 5,5 verwendet. Beispielsweise wird eine Cohn-Fraktion II+III, eine Endfällung venöser oder plazentarer Immunglobuline oder ein Lyophilisat durch Auflösung oder eine eventuell sogar Ethanol enthaltende Immunglobulin-Lösung durch entsprechende Verdünnung und durch Säurezusatz auf pH 4,0 bis 5,0 eingestellt. Diese Lösung aus IgG-Monomeren, IgG-Dimeren, IgG-Trimeren und IgG-Polymeren wird mittels eines Ionenaustauschers aufgetrennt (chromatographiert). Die IgG-Monomere werden mit einem 0,02 bis 0,2 molaren Puffer von pH 4,0 bis 5,5, der zusätzlich 0,05 bis 0,15 mol Kochsalz enthält, eluiert. Dieses Eluat (= geklärter Extrakt) weist lediglich eine schwache antikomplementäre Aktivität auf und wird einer Feinreinigung zugeführt. Die IgG-Aggregate und zwar sowohl die Polymere als auch die Trimere und Dimere bleiben am Ionenaustauscher gebunden und können später mit hochmolarem Puffer abgelöst werden.

Das Dokument DE 27 51 717 C2 offenbart ein Verfahren zur Herstellung eines praktisch von antikomplementärer Aktivität freien und für die intravenöse Verabreichung geeigneten Gamma-Globulins, dessen Antikörperspektrum und Unterklassen-Verteilung gegenüber dem als Ausgangsmaterial verwendeten Plasma praktisch unverändert ist. Hier wird eine Paste von Cohn-Fraktionen II oder II+III oder von diese enthaltendem Plazenta-Extrakt mit pyrogenfreiem Wasser bei einem pH-Wert von 4,9 bis 6 extrahiert. Aus diesem filtrierten Extrakt werden durch stufenweise Fällung mit Polyethylenglykol bis zu einer Konzentration von 4 % und anschließend mit Ethanol bis zu einer Konzentration von 4 bis 12 %, wobei jedesmal die ausgefallenen Verunreinigungen abgetrennt werden, und nach anschließender Steigerung des pH-Wertes auf 7 bis 8,2 durch Erhöhung der Polyethylenglykolkonzentration auf bis zu 12 % oder durch Erhöhung der Ethanolkonzentration auf bis zu 25 % das gewünschte Gamma-Globulin ausgefällt und isoliert. Der Niederschlag wird in einer Lösung aufgenommen, die Human-Albumin, Natriumazetat, Glycin und Mannit enthält und einen pH von 5,1 aufweist. Das applizierbare Präparat kann in Lösung oder als Lyophilisat aufbewahrt werden.

Das Dokument AT 270 068 B1 offenbart ein Verfahren zur Herstellung von Anti-Rh-γ-Globulin. Als Ausgangsmaterial dient menschliches Blutplasma, und die Aufarbeitung erfolgt entsprechend nachstehendem Schema:
- in einer 1. Stufe wird das menschliche Blutplasma auf etwa 1° C gekühlt, und der dabei gebildete Niederschlag wird aus der flüssigen Phase abgetrennt;
- in einer 2. Stufe wird die erhaltene flüssige Phase mit 10,5 bis 10,0 % Methanol versetzt, auf etwa -2° C abgekühlt und auf einen pH-Wert von 7 bis 7,4 und eine Ionenkonzentration von 0,136 gI/l eingestellt, und der dabei gebildete Niederschlag wird aus der flüssigen Phase abgetrennt;
- in einer 3. Stufe wird der in der zweiten Stufe erhaltenen flüssigen Phase 33,25 bis 33,35 % Methanol zugesetzt, die Temperatur wird auf etwa -5° C eingestellt und ferner ein pH-Wert von 6,7 bis 7,1 und eine Ionenkonzentration von 0,081 bis 0,091 gI/l aufrechterhalten, und der dabei gebildete Niederschlag wird aus der flüssigen Phase abgetrennt;
- in einer 4. Stufe wird dem in der dritten Stufe erhaltenen Niederschlag 26,5 bis 26,9 % Methanol zugesetzt, und bei einer Temperatur von etwa -5° C wird ein pH-Wert von 7,0 bis 7,4 und eine Ionenkonzentration von 0,0047 bis 0,0057 gI/l eingestellt, und der dabei gebildete Niederschlag wird aus der flüssigen Phase abgetrennt;
- in einer 5. Stufe wird dem in der vierten Stufe erhaltenen Niederschlag 22,5 bis 22,9 % Methanol zugesetzt, und bei einer Temperatur von etwa -6° C wird ein pH-Wert von 5,0 bis 5,4 und eine Ionenkonzentration von 0,008 bis 0,018 gI/l eingestellt, und der dabei gebildete Niederschlag wird aus der flüssigen Phase abgetrennt; und
- in einer 6. Stufe wird der in der fünften Stufe erhaltenen flüssigen Phase 33,0 bis 33,4 % Methanol zugesetzt, ferner wird eine Temperatur von -6,5° C, ein pH-Wert von 7,0 bis 7,4 und eine Ionenkonzentration von 0,055 bis 0,065 gI/l eingestellt, und der dabei gebildete Niederschlag wird isoliert und gegebenenfalls gefriergetrocknet.

Ersichtlich ist ein vielstufiges Verfahren vorgesehen, das in der 3. Stufe die Bildung eines Niederschlages vorsieht, der in der 4. Stufe durch eine weitere Fällung weiter fraktioniert wird. Insoweit handelt es sich beim bekannten Verfahren um ein mehrstufiges Fällungsverfahren, wobei im Fällungsgemisch u.a. eine bestimmte Methanolkonzentration eingestellt wird. Die höchste Methanolkonzentration beträgt 33,0 bis 33,4 %.

Die U.S.-Patentschrift 4,021,540 offenbart ein Verfahren zur Gewinnung eines Immunglobulin, das gegen Hepatitis-B-Viren wirksam ist. Das Verfahren entspricht weitestgehend dem vorstehend beschriebenen, aus AT 270 068 B1 bekannten Verfahren.

Die bekannten Verfahren sehen mit der chromatographischen Auftrennung oder mit der stufenweisen Polethylenglykol-Ethanol-Fällung oder mit der mehrstufigen Fällung bei unterschiedlichen Methanolkonzentrationen mehrstufige Reinigungsschritte vor. Würde andererseits die Extraktion des Gesamtglobulinniederschlages mit einem schwach sauren Ethanol/ Wasser-Extraktionsmittel unter Cohn-Bedingungen durchgeführt werden, so würden im Extrakt erneut polymere IgG-Aggregate auftreten, und das Produkt könnte nicht oder nur nach weiteren aufwendigen Reinigungsmaßnahmen für eine intravenöse Applikation vorgesehen werden.

Demgegenüber besteht die Aufgabe der vorliegenden Erfindung darin, bei einem Verfahren der eingangs genannten Art aus dem Gesamtglobulin-Niederschlag mit einem preiswerten Lösungsmittel unter definierten, leicht einstellbaren Extraktionsbedingungen in einem einzigen Extraktionsschritt einen geklärten Extrakt zu erzeugen, der direkt oder nach einer wahlweisen zusätzlichen Reinigungsstufe zum applizierbaren Präparat weiterverarbeitet wird.

Ausgehend von einem Verfahren mit den oben angegebenen Maßnahmen ist die erfindungsgemäße Lösung dieser Aufgabe dadurch gekennzeichnet, daß
- der in Stufe (a) erhaltene Gesamtglobulinniederschlag - soweit erforderlich - auf ein Restlösungsmittel eingestellt wird, das neben Wasser 35 bis 40 Vol.-% Methanol enthält und das einen pH von 6,5 bis 7,0 aufweist;
- dieser eingestellte Gesamtglobulinniederschlag in Stufe (b) bei einer Temperatur von 2 bis 8° C mit einem Lösungsmittel extrahiert wird, das neben Wasser 38 bis 42 Vol.-% Methanol enthält; wobei
- das Extraktionsgemisch bei einem pH von 4,7 bis 5,3 und bei einer Methanolkonzentration von 38 bis 42 Vol.-% Methanol im Methanol/Wasser-Gemisch gehalten wird; und
- der dabei erhaltene Extrakt geklärt - wahlweise einer Feinreinigung unterworfen - und nach Stufe (c) weiterverarbeitet wird.

Vorteilhafte Ausgestaltungen und Weiterbildungen des erfindungsgemäßen Verfahrens ergeben sich aus den Unteransprüchen

Mit der erfindungsgemäßen Methanol-Extraktion werden eine Reihe von Vorteilen erzielt. So wird die Nativität des Ausgangs-Gamma-Globulins im Vergleich zu anderen Verfahren nicht nennenswert denaturiert. Die Methanolextraktion als solche führt nicht zu einer nennenswerten Bildung von IgG-Dimeren unter den angegebenen Bedingungen. In dem nach der Methanolextraktion erhaltenen Extrakt treten keine IgG-Trimere und höhere IgG-Polymere auf.

Methanol ist ein preiswertes Lösungs- bzw. Extraktionsmittel. Die Methanolentfernung aus der IgG-Lösung ist unproblematisch, weil zur Einstellung einer isotonischen Salzkonzentration und eines physiologisch verträglichen pH-Wertes typischerweise Dialyseschritte vorgesehen werden, welche auch Methanolspuren aus dem Präparat entfernen.

Die erfindungsgemäß vorgesehenen Extraktionsbedingungen erfordern geringere Energiekosten, weil im Vergleich zu bekannten Verfahren bei höheren Temperaturen gearbeitet werden kann.

Schließlich erfordert die erfindungsgemäße Methanolextraktion eine geringere Anzahl an Verfahrensschritten und liefert eine höhere Ausbeute an applizierbarem IgG-Präparat als vergleichbare bekannte Verfahren.

Als Ausgangsmaterial für die erfindungsgemäße Methanolextraktion dient ein Gesamtglobulinniederschlag, der weniger als 1 Gew.-% IgG-Polymere enthält und der als Restlösungsmittel bereits ein Methanol/Wasser-Gemisch enthält. Dieses Restlösungsmittel soll neben Wasser 35 bis 40 Vol.-% Methanol enthalten und einen pH von 6,5 bis 7,0 aufweisen. Ein, ein anderes Restlösungsmittel enthaltender Gesamtglobulin-niederschlag beispielsweise die Cohn-Fraktionen II und II+III, wird mit einem Methanol/Wasser-Gemisch umgewaschen, das neben Wasser 35 bis 40 Vol.-% Methanol enthält und das einen pH von 6,5 bis 7,0 aufweist. Ein dieses bestimmte Restlösungsmittel enthaltender Gesamtglobulinniederschlag wird nachstehend als eingestellter Gesamtglobulinniederschlag bezeichnet.

Als Ausgangsmaterial zur Bereitstellung eines eingestellten Gesamtglobulinniederschlags dienen bekannte, Gamma-Globulin enthaltende Materialien, wie etwa Überstände der Kryopräzipitatzentrifugation, out-dated-Plasma, die Fraktionen II oder II+III der Cohn'schen Ethanol-Plasma-Fraktionierung, Plazenta-Extrakt, der entsprechende Fraktionen enthält, im Einzelfall gezielt mit Immunkörpern angereichertes Plasma und andere in der Plasmaaufbereitung anfallende Gamma-Gloobulin enthaltende Fraktionen. Die Fällungsbehandlung(en) dieser Ausgangslösungen soll(en) unter schonenden Bedingungen durchgeführt werden, so daß der gebildete Gesamtglobulinniederschlag weniger als 1 Gew.-% IgG-Dimere und IgG-Polymere enthält. Soweit erforderlich, wird der aus diesen Materialien erhaltene Gesamtglobulinniederschlag mit einem Methanol/Wasser-Gemisch umgewaschen, das neben Wasser 35 bis 40 Vol.-% Methanol enthält und das einen pH von 6,5 bis 7,0 aufweist, um einen eingestellten Gesamtglobulinniederschlag zu erhalten.

Vorzugsweise werden bereits die vorstehend genannten IgG-haltigen Ausgangs lösungen mit einem Methanol/Wasser-Fällungsmittel behandelt, um einen Gesamtglobulinniederschlag zu erhalten, der weitgehend frei von IgG-Dimeren und IgG-Polymeren ist. Zur Durchführung dieser Gesamtglobulinfällung wird die Ausgangslösung (Serum, Plasma, Plazenten) mit Pufferlösung auf eine Proteinkonzentration von etwa 4,0 bis 4,5 % und einen pH von etwa 6,4 bis 6,8 eingestellt, und diese eingestellte Ausgangslösung wird mit reinem Methanol oder mit einer Methanol/Wasser-Lösung versetzt, die neben Wasser etwa 60 bis 99 Vol.-% Methanol enthält, wobei das Methanol oder die Methanol/Wasser-Lösung eine Temperatur von -8° C bis -25° C aufweist. Die eingestellte Ausgangslösung wird mit einer solchen Menge Methanol oder Methanol/ Wasser-Lösung versetzt, die zu einem Methanolgehalt von etwa 40 Vol.-% im Restlösungsmittel der Gesamtglobulinfällung führt. Der Niederschlag wird abgetrennt und enthält bereits das für die Weiterverarbeitung vorgesehene, bestimmte Restlösungsmittel.

Der eingestellte Gesamtglobulinniederschlag wird der erfindungsgemäßen Methanolextraktion zugeführt. Hierbei wird der eingestellte Gesamtglobulinniederschlag bei einer Temperatur von 2 bis 8° C mit einem Lösungsmittel extrahiert, das neben Wasser 38 bis 42 Vol.-% Methanol enthält. Vorzugsweise enthält das Lösungsmittel neben Wasser etwa 40 Vol.-% Methanol. Das Extraktionsgemisch wird bei einem pH von 4,7 bis 5,3 und bei einer Methanolkonzentration von 38 bis 42 Vol.-% Methanol im Methanol/Wasser-Gemisch gehalten.

Beträgt die Methanolkonzentration des Methanol/Wasser-Gemisches im Extraktionsgemisch weniger als 38 Vol.-% Methanol, so gehen bereits Spuren von Begleitproteinen in Lösung und das Produkt wird unreiner. Beträgt andererseits die Methanolkonzentration im Methanol/Wasser-Gemisch des Extraktionsgemisches mehr als 42 Vol.-% Methanol, so geht das Gamma-Globulin nicht ausreichend in Lösung und die Ausbeuten sinken schlagartig. Vorzugsweise wird bei einer Methanolkonzentration von etwa 40 Vol.-% im Methanol/Wasser-Gemisch des Extraktionsgemisches gearbeitet. Hier wird ein besonders reines Produkt erhalten und das im Gesamtglobulinniederschlag enthaltene Gamma-Globulin löst sich vollständig.

Die erfindungsgemäße Methanolextraktion wird bei einer Temperatur zwischen +2 bis +8° C durchgeführt. Es wäre möglich, auch bei tieferen Temperaturen zu arbeiten, wobei jedoch die Methanolkonzentration an die dann gegebene Löslichkeit von Gamma-Globulin angepaßt werden müßte. Abgesehen von Sonderfällen bringt ein Arbeiten bei tieferen Temperaturen keine Vorteile, erfordert jedoch einen höheren Energiebedarf. Vorzugsweise wird die erfindungsgemäße Methanolextraktion bei einer Temperatur von etwa +4° C durchgeführt.

Weiterhin wird im Extraktionsgemisch ein pH zwischen 4,7 und 5,3 aufrecht erhalten. Bei einem pH unterhalb 4,7 tritt unter diesen Extraktionsbedingungen eine Denaturierung des Gamma-Globulins auf. Bei einem pH oberhalb 5,3 wird eine schleichende Ausbeuteabnahme beobachtet. Vorzugsweise wird bei einem pH zwischen 5,1 und 5,2 im Extraktionsgemisch gearbeitet. Hier wird eine optimale Ausbeute und Reinheit des Gamma-Globulins erhalten.

Die laufende Überwachung des pH-Wertes des Extraktionsgemisches kann mit Hilfe von Wasserstoffelektroden erfolgen. Zur pH-Einstellung wird der Gesamtglobulinniederschlag direkt mit 0,01 m Na-Azetat-Puffer 4,7 in Lösung gebracht. Jegliche Eintropfdenaturierung und/oder Polymerbildung wird vermieden.

Vorzugsweise wird 1 Gewichtsteil Gesamtglobulinniederschlag mit etwa 2 bis 6 Gewichtsteilen Methanol/Wasser-Gemisch extrahiert. Besonders bevorzugt wird 1 Gewichtsteil Gesamtglobulinniederschlag mit etwa 4 Gewichtsteilen Lösungsmittel extrahiert. Es wird eine optimale Gamma-Globulinausbeute erhalten.

Je nach Art und Zusammensetzung des Gesamtglobulinniederschlags gehen unter diesen Extraktionsbedingungen etwa 1/3 der Eiweißmenge in Lösung. Der restliche Anteil wird verworfen oder kann zu anderen Produkten aufgearbeitet werden. Der gebildete Extrakt wird durch Filtration und/oder Zentrifugation von dem verbleibenden Rest abgetrennt. Es wird ein geklärter Extrakt erhalten, der abhängig von der Restfeuchte des Gesamtglobulinniederschlags zwischen etwa 1,0 bis 3,0 Gew.-% hochreines Gamma-Globulin (Reinheit über 95 %) enthält. Dieser geklärte Extrakt kann bei Bedarf wahlweise einer zusätzlichen Feinreinigung unterworfen werden, oder dieser geklärte Extrakt wird direkt der Weiterverarbeitung zum applizierbaren Präparat zugeführt.

Die für Fachleute überraschende Reinheit der erfindungsgemäß erhältlichen IgG-Lösung wird auch durch die Figuren bestätigt; es zeigen:

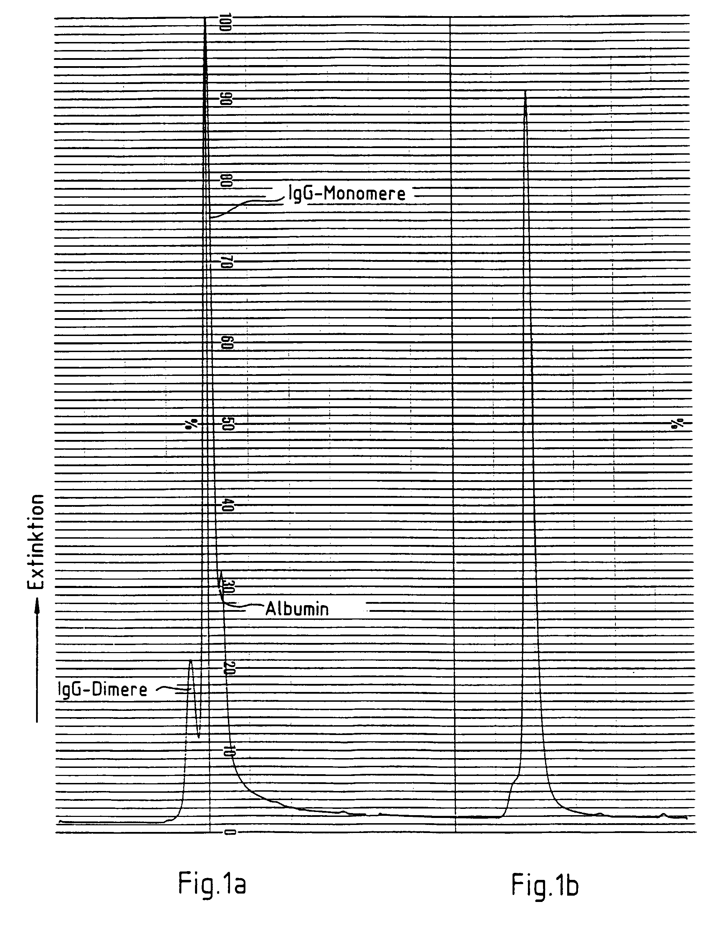

- Figur 1a: ein HPLC-Chromatogramm eines handelsüblich zugänglichen, applizierbaren IgG-Präparates, das nach Cohn-Fraktionierung und mehreren Reinigungsschritten erhalten wurde; neben dem Hauptpeak des IgG-Monomeren sind deutlich die Peaks für IgG-Dimere und für Albumin zu erkennen, das dem Präparat als Stabilisator zugesetzt ist; und
- Figur 1b: das unter analogen Bedingungen erhaltene HPLC-Chromatogramm eines geklärten IgG-Extraktes, der nach einmaliger, erfindungsgemäßer Methanolextraktion aus einem, weniger als 1 Gew.-% IgG-Polymere enthaltenden Gesamtglobulinniederschlag erhalten wurde. Es ist deutlich zu erkennen, daß die für die IgG-Dimeren charakteristische Absorption lediglich als schwache Schulter ausgeprägt ist, was bestätigt, daß dieser erfindungsgemäß erzeugte IgG-Extrakt im wesentlichen vollständig aus IgG-Monomeren besteht und IgG-Dimere lediglich in Spuren vorhanden sind.

Für Fachleute ist überraschend, daß eine Methanolextraktion unter den erfindungsgemäß vorgesehenen Bedingungen keinerlei Denaturierung der Immunglobuline hervorruft und daß bereits eine einstufige Methanolextraktion ein derart hochreines Präparat liefert.

Die Bedingungen der erfindungsgemäßen Methanolextraktion unterscheiden sich eindeutig von den typischen Bedingungen der Cohn'schen Ethanol-Franktionierung. Typischerweise wird nach Cohn die Gamma-Globulin enthaltende Ausgangslösung bei 0° C und pH 5,8 durch Ethanolzugabe auf einen Ethanolgehalt von 9 Vol.-% Ethanol gebracht. Nach Einstellung stabiler Bedingungen wird auf -5° C abgekühlt und es erfolgt eine Zugabe von tiefgekühltem (-8° C) Ethanol bis zu einer Ethanolkonzentration von 19 Vol.-% Ethanol im Fällungsgemisch. Es wird weiter bis auf -8° C abgekühlt. Der gebildete Niederschlag wird abgetrennt und durch Zentrifugation oder Filtration von überschüssigem Lösungsmittel befreit. Zur Weiterverarbeitung und Lösung des Gamma-Globulins kann dieser Niederschlag mit einem wässrigen Extraktionsmittel behandelt werden, das durch Zugabe von Na-Azetat und Essigssäure auf einen pH von etwa 4,6 eingestellt ist. Unter diesen Cohn'schen Bedingungen kann ein intravenös applizierbares Präparat nicht erhalten werden; vielmehr sind weitere Reinigungsschritte erforderlich, beispielsweise mit Hilfe einer stufenweisen PEG-Zugabe und Abtrennung der jeweils gebildeten Niederschläge.

Würde unter den oben dargelegten Cohn'schen Fällungsbedingungen anstelle von Ethanol mit Methanol als organischem Fällungsmittel gearbeitet werden, so könnte eine nennenswerte Ausbeute an Gamma-Globulin nicht erhalten werden. Würde andererseits unter den Bedingungen der erfindungsgemäßen Methanolextraktion anstelle von Methanol mit Ethanol gearbeitet werden, so würde das zur Denaturierung führen, und eine verwertbare Ausbeute an Gamma-Globulin könnte ebenfalls nicht erzielt werden. Unter Beibehaltung der restlichen Bedingungen kann Methanol nicht durch Ethanol ersetzt werden und umgekehrt.

Der nach der erfindungsgemäßen Methanolextraktion erhaltene, klar filtrierte Extrakt wird bei Bedarf wahlweise einer Feinreinigung unterworfen. Die Feinreinigung kann mit Hilfe bekannter Maßnahmen durchgeführt werden, beispielsweise durch Adsorption der unerwünschten Begleitkomponenten an Kieselsäure oder an andere Mineralien vom Typ der Alumosilikate. Gute Ergebnisse wurden beispielsweise durch Adsorption an den Anionenaustauscher "SEPHADEX DEAE-A 50" erzielt. Die Feinreinigung kann einstufig durchgeführt werden, oder mehrstufig durch Wiederholung des gleichen Reinigungsschrittes oder durch aufeinanderfolgende Anwendung verschiedener Reinigungsschritte. Vorzugsweise ist eine Feinreinigung vorgesehen, die als einen ersten Reinigungsschritt eine Wiederholung der oben beschriebenen Methanol-extraktion vorsieht.

Bei diesem vorzugsweise vorgesehenen Feinreinigungsschritt erfolgt eine IgG-Ausfällung durch pH-Anhebung in methanolischer Lösung und daraufhin erneut eine Methanolextraktion des Niederschlags. Von der vorausgegangenen Methanolextraktion her weist der klar filtrierte Extrakt einen pH von 5,1 auf. Unter Konstanthaltung des Methanolgehaltes bei etwa 40 Vol.-% Methanol wird der pH des Extraktes auf 6,5 bis 7,3 angehoben. Besonders bevorzugt wird der pH auf 6,9 bis 7,0 angehoben. Diese pH-Anhebung kann mit bekannten Pufferlösungen erfolgen; vorzugsweise wird 0,5 m NaHCO₃-Lösung zugesetzt. Unter den gewählten Bedingungen für Temperatur und Methanolgehalt wird lediglich das monomere Immunglobulin ausgefällt, ohne daß die in Spuren vorhandenen Begleitproteine mitgerissen werden. Der gebildete Niederschlag wird abgetrennt, beispielsweise durch Zentrifugieren oder Filtrieren. Der erhaltene Niederschlag wird mit dem Fällungsmittel (40 Vol.-% Methanol, pH 6,9 bis 7,0) gewaschen. Das gewaschene Präzipitat wird erneut der erfindungsgemäßen Methanolextraktion zugeführt. Die Methanolextraktion erfolgt unter den oben angegebenen Bedingungen, d.h., bei einer Temperatur von etwa 4° C mit einem 40 Vol.-% Methanol enthaltenden Lösnungsmittel, das einen pH von etwa 4,7 aufweist. Abweichend kann eine geringere Menge Lösungsmittel eingesetzt werden, um die IgG-Verluste in dem im Niederschlag verbleibenden Restlösungsmittel möglichst gering zu halten. Vorzugsweise wird bei dieser erneuten Methanolextraktion im Verlauf der Feinreinigung 1 Gewichtsteil Niederschlag mit etwa 3 Gewichtsteilen Lösungsmittel behandelt. Der danach erhaltene Extrakt wird klar filtriert. Die klar filtrierte IgG-Lösung ist praktisch frei von unerwünschten Begleitproteinen und wird der Dialyse oder den Dialysen zur Methanolentfernung, Einstellung eines physiologisch verträglichen pH-Wertes und einer isotonischen Salzkonzentration zugeführt. Diese Dialyseschritte werden in bekannter Weise durchgeführt. Lediglich im Einzelfall und bei Bedarf kann im Anschluß an die zweite Methanolextraktion im Verlauf der Feinreinigung noch eine weitere Feinreinigungsstufe, beispielsweise mit einem Anionenaustauscher vorgesehen werden. Dies kann beispielsweise dann angezeigt sein, wenn der Ausgangs-Gesamtglobulinniederschlag einen unüblich hohen Anteil an dimeren und polymeren IgG-Aggregaten enthalten hat.

Die Weiterverarbeitung des nach Stufe (b) erhaltenen geklärten Extraktes oder der nach der Feinreinigung erhaltenen IgG-Lösung zum applizierbaren Präparat einschließlich Einstellung eines physiologisch verträglichen pH-Wertes, Zugabe von Stabilisierungsmittel(n), Einstellung der gewünschten Proteinkonzentration, Sterilisation und ggf. Virusinaktivierung/-Elimination - gemäß Stufe (c) - erfolgt mit Hilfe üblicher, in der Fachwelt bekannter Maßnahmen. Beispielsweise kann zur Einstellung eines physiologisch verträglichen pH-Wertes eine Dialyse mit 0,05 m Na-Citrat-Puffer pH 7,0 vorgesehen werden. Als Stabilisierungsmittel haben sich beispielsweise Glycin, Glukose und/oder Mannit bewährt. Beispielsweise kann zur Stabilisierung und zur Einstellung einer isotonischen Salzkonzentration eine Enddialyse mit einer Lösung von 3 % Glukose und 0,6 % NaCl vorgesehen werden. Zur Sterilisation kann eine Sterilfiltration vorgesehen werden. Auch eine Virusinaktivierung oder Viruselimination kann in bekannter Weise erfolgen. Zweckmäßigerweise wird das applizierbare Präparat auf einen IgG-Gehalt von 5 Gew.-% eingestellt.

Die nachstehenden Beispiele dienen zur weiteren Erläuterung der Erfindung ohne diese einzuschränken.

### Beispiel 1:

Als Ausgangsmaterial für die erfindungsgemäße Herstellung von nativem, intravenös verabreichbarem, stabilisiertem Immunglobulin (IgG) werden Überstände der Kryopräzipitatzentrifugation, out-dated-Plasma oder andere in der Plasmaaufarbeitung anfallende, Gamma-Globulin enthaltende Fraktionen eingesetzt. In jedem Falle wird von einer Lösung ausgegangen, der 0,05 m Na-Acetat-Puffer pH 4,7 zugesetzt wird, um die Proteinkonzentration auf 4,3 Gew.-% einzustellen. Nach Zusatz des Puffers weist die Mischung einen pH von 6,4 bis 6,8 auf. Das Plasma-Puffer-Gemisch wird auf eine Temperatur von etwa 4° C gebracht.

Zur Erzeugung eines Gesamtglobulinniederschlags wird dieses, bei 4° C gehaltene Plasma-Puffer-Gemisch mit einer tiefgekühlten, wässrigen Methanol-Lösung versetzt, die neben Wasser etwa 65 Vol.-% Methanol enthält und eine Temperatur von -8° C bis -25° C aufweist. Unter diesen Bedingungen wird jegliche Eintropfdenaturierung vermieden. Die tiefgekühlte wässrige Methanol-Lösung wird so lange zugesetzt, bis im Fällungsgemisch eine Methanolkonzentration in der Wasser/Methanol-Lösung von 40 Vol.-% Methanol erreicht ist. Das gebildete Präzipitat wird durch Zentrifugation oder Filtration abgetrennt und dient als Gesamtglobulinniederschlag zur erfindungsgemäßen Herstellung eines intravenös applizierbaren IgG-Präparates. Aufgrund der vorausgegangenen Methanolfällung enthält dieser Gesamtglobulinniederschlag ein Restlösungsmittel, das neben Wasser 35 bis 40 Vol.-% Methanol enthält und das einen pH von 6,5 bis 7,0 aufweist.

Dieser, das bestimmte Restlösungsmittel enthaltende Gesamtglobulinniederschlag wird der erfindungsgemäßen Methanol-extraktion zugeführt. Die Extraktion des Niederschlages erfolgt mit einem Lösungsmittel, das aus 40 Vol.-% Methanol und wässrigem 0,01 m Na-Acetat-Puffer mit einem pH von 4,7 besteht. 1 Gewichtsteil Gesamtglobulinniederschlag wird mit 4 Gewichtsteilen Lösungsmittel extrahiert. Das Extraktionsgemisch wird bei einem pH von 5,1 und bei einer Methanolkonzentration von etwa 40 Vol.-% Methanol im Methanol/Wasser-Gemisch gehalten. Die im Lösungsmittel unlöslichen Anteile werden abgetrennt und der gebildete Extrakt wird klar filtriert. Eine Probe dieses geklärten Extraktes liefert das HPLC-Chromatogramm gemäß Figur 1b.

Zur wahlweisen Feinreinigung wird dieser geklärte Extrakt erneut umgefällt. Hierzu wird 0,5 m NaHCO₃-Lösung zugesetzt, um den pH auf 6,9 bis 7,0 anzuheben; hierbei wird der Methanolgehalt des Gemisches konstant gehalten. Der gebildete IgG-Niederschlag wird mittels Zentrifugation oder Filtration abgetrennt. Der erhaltene Niederschlag wird unter Fällungsbedingungen gewaschen. Das gewaschene Präzipitat wird erneut mit dem Methanol/Wasser-Lösungsmittel unter den vorstehend angegebenen Bedingungen extrahiert, wobei 1 Gewichtsteil IgG-Präzipitat mit 3 Gewichtsteilen Lösungsmittel behandelt werden. Der danach erhaltene Extrakt wird klar filtriert.

Die nach Klarfiltration und wahlweiser Feinreinigung anfallende IgG-Lösung wird zum applizierbaren Präparat weiterverarbeitet. Die Dialyse der so erhaltenen IgG-Lösung erfolgt zunächst mit 0,05 m Na-Citrat-Puffer pH 7,0. Die Enddialyse erfolgt mit einer Lösung von 3 % Glukose und 0,6 % NaCl. Die resultierende IgG-Lösung wird abschließend isoosmotisch und auf einen Proteingehalt von 5 % eingestellt. Sämtliche Fällungsund Separationsschritte werden bei einer Temperatur von 4° C ausgeführt. Das HPLC-Chromatogramm des so erhaltenen, applizierbaren IgG-Präparates zeigt einen Gehalt an 99 % IgG-Monomeren und lediglich Spuren von IgG-Dimeren.

### Beispiel 2:

Als Ausgangsmaterial zur erfindungsgemäßen Herstellung von nativem, intravenös verabreichbarem Immunglobulin dient das Präzipitat der Fraktion II+III der Cohn'schen Ethanol-Plasma-Fraktionierung, sofern dieses weitgehend frei von IgG-Polymeren (weniger als 1 Gew.-% ) ist. Aufgrund der Cohn'schen Fraktionierungsbedingungen enthält dieser Gesamtglobulinniederschlag als Restlösungsmittel ein Ethanol/Wasser-Gemisch. Dieser ethanolhaltige Gesamtglobulinniederschlag wird bei einer Temperatur von 4° C mit einem Methanol/Wasser-Gemisch umgewaschen, das neben Wasser 40 Vol.-% Methanol enthält und einen pH von 6,7 bis 7,0 aufweist; zur pH-Einstellung dient 0,01 m Na-Acetat-Puffer. 1 kg ethanolhaltiger Gesamtglobulinniederschlag wird in 4 1 Methanol/Wasser-Gemisch aufgeschlämmt. Daraufhin wird der Niederschlag erneut durch Filtration oder Zentrifugation abgetrennt, wobei ein eingestellter Gesamtglobulinniederschlag erhalten wird, dessen Restlösungsmittel neben Wasser 35 bis 40 Vol.-% Methanol enthält und einen pH von 6,5 bis 7,0 aufweist.

Dieser eingestellte Gesamtglobulinniederschlag wird daraufhin der erfindungsgemäßen Methanolextraktion zugeführt, wie das in Beispiel 1 beschrieben ist. Das Lösungsmittel besteht aus 0,01 m Na-Acetat-Puffer und 40 Vol.-% Methanol und weist einen pH von 4,7 auf. 1 kg eingestellter Gesamtglobulinniederschlag wird mit 4 1 Lösungs- bzw. Extraktionsmittel behandelt. Der pH-Wert des Extraktionsgemisches wird bei 5,1 gehalten. Der erhaltene Extrakt wird klar filtriert. Die weitere Verarbeitung zum applizierbaren Präparat erfolgt in gleicher Weise wie oben in Beispiel 1 angegeben. Das HPLC-Chromatogramm des applizierbaren Präparates zeigt 99 % IgG-Monomere und lediglich Spuren von Dimeren.

### Beispiel 3:

Die erfindungsgemäße Methanolextraktion des Gesamtglobulinniederschlags erfolgt in gleicher Weise wie in Beispiel 1 angegeben. Der nach der Klarfiltration erhaltene saure Extrakt wird der Feinreinigung zugeführt. In diesem Falle wird dieser saure Extrakt zunächst zur Entfernung von Methanol gegen 0,05 m Citrat-Phosphat-Puffer, pH 7,0 dialysiert und danach gegen eine 0,1 m NaCl-Lösung, pH 7,0 dialysiert, und das erhaltene Material auf eine Konzentration von 6 bis 8 % Eiweiß eingestellt. Dieses Material wird mit dem Anionenaustauscher SEPHADEX DEAE-A 50 behandelt, der vorher auf oben genannte Milieubedingungen äquilibriert und autoklaviert worden ist. Etwa 1 1 konzentrierter Extrakt wird mit 2 g SEPHDEX DEAE-A 50 behandelt. Hierdurch werden Spurenbegleitkomponenten des IgG an das Austauscherharz adsorbiert und auf diesem Wege entfernt. Das beladene Austauscherharz wird abgetrennt. Daraufhin erfolgt die Stabilisierung und Einstellung der IgG-Lösung zum applizierbaren Präparat.

Die erfindungsgemäß erhältlichen, nativen, intravenös applizierbaren IgG-Präparate sind insbesondere zum Einsatz im Humanbereich bestimmt. Wegen der geringen Verluste und deshalb hohen Ausbeute ist das Verfahren auch zur Aufarbeitung von hyperimmunisiertem Plasma gut geeignet. Darüber hinaus können erfindungsgemäß erhältliche IgG-Präparate auch im veterinärmedizinischen Bereich eingesetzt werden, beispielsweise zur Behandlung von Pferd, Rind, Schwein oder Hund, sofern von homologen Ausgangsmaterialien ausgegangen wird.

## Patentansprüche

1. Verfahren zur Herstellung von nativem, intravenös verabreichbarem, stabilisiertem Immunglobulin (IgG),
wobei bei einer Temperatur unterhalb 8° C
a) unter Maßnahmen zur schonenden Gesamtglobulinfällung aus Serum, Plasma oder Plazenten ein IgG enthaltender Gesamtglobulinniederschlag ausgefällt und abgetrennt wird, der weniger als 1 Gew.-% Polymere enthält,
b) dieser Gesamtglobulinniederschlag bei schwach saurem pH mit einer schwach ionischen, wässrigen, alkoholischen Lösung behandelt wird, und
c) die danach erhaltene IgG-Lösung vom alkoholischen Lösungsmittel befreit, auf eine gewünschte Proteinkonzentration sowie isotonische Salzkonzentration und einen physiologisch verträglichen pH-Wert eingestellt wird und durch Zugabe von Stabilisierungsmittel(n), Sterilisation und gegebenenfalls Virusinaktivierung/ -elimination, zu einem applizierbaren Präparat weiterverarbeitet wird,
dadurch gekennzeichnet, daß
der in Stufe (a) erhaltene Gesamtglobulinniederschlag - soweit erforderlich - auf ein Restlösungsmittel eingestellt wird, das neben Wasser 35 bis 40 Vol.-% Methanol enthält und das einen pH von 6,5 bis 7,0 aufweist;
dieser eingestellte Gesamtglobulinniederschlag in Stufe (b) bei einer Temperatur von 2 bis 8° C mit einem Lösungsmittel extrahiert wird,
das neben Wasser 38 bis 42 Vol.-% Methanol enthält; wobei das Extraktionsgemisch bei einem pH von 4,7 bis 5,3 und bei einer Methanolkonzentration von 38 bis 42 Vol.-% Methanol im Methanol/ Wasser-Gemisch gehalten wird; und
der dabei erhaltene Extrakt geklärt, - wahlweise einer Feinreinigung unterworfen - und nach Stufe (c) weiterverarbeitet wird.

2. Verfahren nach Anspruch 1,
dadurch gekennzeichnet, daß
zur Feinreinigung der pH des geklärten Extraktes auf 6,5 bis 7,0 angehoben wird;
der dabei gebildete Niederschlag separiert wird; und
dieser separierte Niederschlag erneut bei einer Temperatur von 2 bis 8° C mit einem Lösungsmittel extrahiert wird, das neben Wasser 38 bis 42 Vol.-% Methanol enthält; wobei
das Extraktionsgemisch bei einem pH von 4,7 bis 5,3 und bei einer Methanolkonzentration von 38 bis 42 Vol.-% Methanol im Methanol/ Wasser-Gemisch gehalten wird.

3. Verfahren nach Anspruch 2,
dadurch gekennzeichnet, daß
der im Verlauf der Feinreinigung separierte Niederschlag mit einer solchen Menge Lösungsmittel extrahiert wird, um eine IgG-Lösung zu erhalten, die eine IgG-Konzentration größer 5 %, vorzugsweise eine IgG-Konzentration von 6 bis 9 % aufweist.

4. Verfahren nach einem der Ansprüche 1 bis 3,
dadurch gekennzeichnet, daß
ein Gewichtsanteil eingestellter Gesamtglobulinniederschlag mit etwa 2 bis 6 Gewichtsteilen Lösungsmittel extrahiert wird.

5. Verfahren nach einem der Ansprüche 1 bis 4,
dadurch gekennzeichnet, daß
das Lösungsmittel neben Wasser etwa 40 Vol.-% Methanol enthält.

6. Verfahren nach einem der Ansprüche 1 bis 5,
dadurch gekennzeichnet, daß
das Lösungsmittel einen pH von 4,7 bis 5,3 aufweist.

7. Verfahren nach Anspruch 6,
dadurch gekennzeichnet, daß
die pH-Einstellung des Lösungsmittels mit Na-Azetat-Pufferlösung erfolgt.

8. Verfahren nach einem der Ansprüche 1 bis 7,
dadurch gekennzeichnet, daß
das Lösungsmittel eine 0,0005 bis 0,02 molare Ionenkonzentration aufweist.

9. Verfahren nach einem der Ansprüche 1 bis 8,
dadurch gekennzeichnet, daß
das Extraktionsgemisch bei einem pH von 5,1 bis 5,2 gehalten wird.

10. Verfahren nach einem der Ansprüche 1 bis 9,
dadurch gekennzeichnet, daß
zur Durchführung der Gesamtglobulinfällung nach Stufe (a) die Ausgangslösung (Serum, Plasma, Plazenten) mit Pufferlösung auf eine Proteinkonzentration von etwa 4,0 bis 4,5 % und auf einen pH von etwa 6,4 bis 6,8 eingestellt wird;
diese eingestellte Ausgangslösung mit reinem Methanol oder mit einer Methanol/Wasser-Lösung versetzt wird, die neben Wasser etwa 60 bis 99 Vol-% Methanol enthält, wobei das Methanol oder die Methanol/ Wasser-Lösung eine Temperatur von -8° C bis -25° C aufweist; und
die eingestellte Ausgangslösung mit einer solchen Menge Methanol oder Methanol/Wasser-Lösung versetzt wird, die zu einem Methanolgehalt von etwa 40 Vol.-% im Restlösungsmittel der Gesamtglobulinfällung führt.

11. Verfahren nach einem der Ansprüche 1 bis 10,
dadurch gekennzeichnet, daß
in Stufe (c) das alkoholische Lösungsmittel durch Dialyse oder durch Anionenaustauscher-Behandlung entfernt wird.

## Claims

1. A method of manufacturing
a native, intravenously administerable and stabilized immunoglobulin (IgG),
comprising the following steps:
wherein at a temperature below 8° C
a) using mild conditions when precipitating and isolating an IgG containing total globulin precipitate comprising less than 1 % by weight polymer from serum, plasma or placental sources;
b) treating said total globulin precipitate with an ionically weak aqueous, alcoholic solution at a weak acidic pH, in order to obtain an IgG solution;
c) removing from the thus obtained IgG solution the alcoholic solvent and adjusting the thus obtained IgG solution to each a selected protein concentration, an isotonic salt concentration and a physiologically applicable pH value and further processing the thus obtained and adjusted IgG solution by adding at least one stabilising agent and by optionally effecting a virus inactivation and/or virus elimination treatment in order to obtain an administerable IgG preparation,
**characterized in** that
if required - adjusting the total globulin precipitate as obtained according to step (a) to a residual solvent containing water and methanol at a methanol concentration of from 35 to 40 % by volume and comprising a pH of from 6.5 to 7.0;
extracting the thus adjusted total globulin precipitate during step (b) at a temperature of from 2 to 8° C by means of a solvent containing water and methanol at a methanol concentration of from 38 to 42 % by volume;
wherein maintaining said extraction mixture at a pH of from 4.7 to 5.3 and at a methanol concentration of from 38 to 42 % by volume within the methanol/water mixture; and
subjecting the thus obtained extract to a clarification treatment; and
optionally subjecting the thus obtained clarified extract to a fine purifying treatment; and
subjecting the thus obtained extract to the further processing treatment according to step (c).

2. The method according to claim 1,
**characterized in** that
subjecting the clarified extract to a fine purifying treatment;
increasing a pH of the clarified extract to a value within a range of from 6.5 to 7.0 when effecting said fine purifying treatment;
isolating a precipitate being formed during said fine purifying treatment; and
renewed extracting said isolated precipitate at a temperature of from 2 to 8° C by means of a solvent containing water and methanol at a methanol concentration of from 38 to 42 % by volume;
wherein maintaining the extraction mixture at a pH of from 4.7 to 5.3 and at a methanol concentration of from 38 to 42 % by volume within the water/methanol mixture.

3. The method according to claim 2,
**characterized in** that
extracting the isolated precipitate being formed during the fine purifying treatment by means of said solvent in such an amount which will provide an IgG solution having an IgG concentration higher than 5 %, preferably having an IgG concentration of from 6 to 9 %.

4. The method according to anyone of the claims 1 to 3,
**characterized in** that
extracting 1 part per weight adjusted total globulin precipitate by means of 2 to 6 parts per weight of said solvent.

5. The method according to anyone of the claims 1 to 4,
**characterized in** that
said solvent contains water and methanol in a methanol concentration of about 40 % by volume.

6. The method according to anyone of the claims 1 to 5,
**characterized in** that
said solvent comprises a pH of from 4.7 to 5.3.

7. The method according to claim 6,
**characterized in** that
adjusting a pH of said solvent by means of a sodium acetate buffer solution.

8. The method according to anyone of the claims 1 to 7,
**characterized in** that
said solvent comprises a molar ionic concentration of from 0.0005 to 0.02.

9. The method according to anyone of claims 1 to 8,
**characterized in** that
maintaining said extraction mixture at a pH of from 5.1 to 5.2

10. The method according to anyone of the claims 1 to 9,
**characterized in** that
adjusting the starting solution (serum, plasma, placental sources) to a protein concentration of about 4.0 to about 4.5 % and to a pH of from about 6.4 to about 6.8 by means of a buffer solution when precipitating the total globulin precipitate according to step (a);
adding to the thus adjusted starting solution pure methanol or a methanol/water mixture containing water and methanol at a methanol concentration of from about 60 to about 99 % by volume, wherein said pure methanol or said methanol/water mixture each comprising a temperature of from minus 8° C to minus 25° C; and
adding said pure methanol or said water/methanol mixture to said adjusted starting solution in such an amount which will provide a methanol content of about 40 % per volume within the residual solvent of the total globulin precipitate.

11. The method according to anyone of the claims 1 to 10,
**characterized in** that
removing the alcoholic solvent in step (c) by a dialysing treatment or by an anion exchange treatment.

## Revendications

1. Procédé de préparation de
immunoglobuline (IgG), native, administrable par voie intraveineuse et stabilisée,
dans lequel en opérant à une température inférieure à 8°C,
a) on précipite, en prenant des mesures pour la précipitation ménagée de l'ensemble des globulines, dans du sérum, dans du plasma ou dans du placenta, un précipité de l'ensemble des globulines contenant de l'IgG et renfermant moins de 1 % en poids de polymères, et on le sépare
b) on traite ce précipité de l'ensemble des globulines à un pH faiblement acide par une solution aqueuse alcoolique faiblement ionique et
c) on débarrasse la solution d'lgG ainsi obtenue du solvant alcoolique, on la règle à une concentration protéinique souhaitée ainsi qu'à une concentration de sel souhaité et un pH acceptable physiologiquement et, par addition d'agent (s) de stabilisation, par stérilisation et, le cas échéant, par activation/élimination de virus, on la transforme en une application applicable,
caractérisé en ce que
on fait en sorte que le précipité de l'ensemble des globulines obtenues au stade (a) ait, dans la mesure où c'est nécessaire, un solvant restant qui renferme, outre de l'eau, de 35 à 40 % en volume de méthanol et qui a un pH de 6,5 à 7,0;
on fait subir à ce précipité de l'ensemble des globulines ainsi réglé, au stade (b), en opérant à une température de 2 à 8°C, une extraction par un solvant qui renferme, outre de l'eau, de 38 à 42 % en volume de méthanol; le mélange d'extraction étant maintenu à un pH de 4,7 à 5,3 et à une concentration de méthanol de 38 à 42 % en volume de méthanol du mélange méthanol/eau; et
on purifie l'extrait ainsi obtenu, on le soumet éventuellement à une purification poussée et on le transforme suivant le stade (c).

2. Procédé suivant la revendication 2,
caractérisé en ce que
pour la purification poussée, on porte le pH de l'extrait purifié entre 6,5 et 7,0;
on sépare le précipité ainsi formé; et
on fait subir à nouveau au précipité séparé une extraction à une température de 2 à 8°C par un solvant qui renferme, outre de l'eau, de 38 à 42 % en volume de méthanol;
le mélange d'extraction étant maintenu à un pH de 4,7 à 5,3 et à une concentration de méthanol de 38 à 42 % en volume de méthanol du mélange méthanol/eau.

3. Procédé suivant la revendication 2,
caractérisé en ce que
on fait subir au précipité séparé, lors de la purification poussée, une extraction par une quantité de solvant telle que l'on obtienne une solution d'lgG qui a une concentration d'lgG supérieure à 5 % et, de préférence, une concentration d'lgG de 6 à 9 %.

4. Procédé suivant l'une des revendications 1 à 4,
caractérisé en ce que
on fait subir à une partie en poids du précipité réglé de l'ensemble des globulines une extraction par environ 2 à 6 parties en poids de solvant.

5. Procédé suivant l'une des revendications 1 à 4,
caractérisé en ce que
le solvant renferme, outre de l'eau, environ 40 % en volume de méthanol.

6. Procédé suivant l'une des revendications 1 à 5,
caractérisé en ce que
le solvant a un pH de 4,7 à 5,3.

7. Procédé suivant la revendication 6,
caractérisé en ce que
l'on effectue le réglage du pH du solvant par une solution tampon à l'acétate de sodium.

8. Procédé suivant l'une des revendications 1 à 7,
caractérisé en ce que
le solvant a une concentration ionique en mole de 0,0005 à 0,02.

9. Procédé suivant l'une des revendications 1 à 8,
caractérisé en ce que
l'on maintient le mélange d'extraction à un pH de 5,1 à 5,2.

10. Procédé suivant l'une des revendications 1 à 9,
caractérisé en ce que
pour effectuer la précipitation de l'ensemble des globulines suivant le stade (a), on met la solution de départ (sérum, plasma, placenta) par une solution tampon à une concentration de protéines de 4,0 à 4,5 % environ et à un pH de 6,4 à 6,8 environ;
on mélange cette solution de départ, mise à la concentration de protéines et au pH voulus, à du méthanol pur ou à une solution constituée d'un mélange de méthanol et d'eau, qui renferme, outre de l'eau, de 60 à 99 % en volume de méthanol, le méthanol ou la solution de méthanol et d'eau ayant une température de -8°C à -25°C; et on mélange la solution de départ, dont on a réglé la concentration en protéine et le pH, à une quantité telle de méthanol ou de solution de méthanol et d'eau que l'on obtient une teneur en méthanol de 40 % en volume environ dans le solvant restant de la précipitation de l'ensemble des globulines.

11. Procédé suivant l'une des revendications 1 à 10,
caractérisé en ce que
au stade (c), on élimine le solvant alcoolique par dialyse ou par un traitement par échange d'anions.
